# EUROPEAN PATENT SPECIFICATION

(11) **EP 3 937 115 B1**
(45) Date of publication and mention of the grant of the patent: **23.09.2026**
(21) Application number: 20918127.0
(22) Date of filing: 27.05.2020
(51) Int. Cl.: G06Q 50/10, G01N 33/00, G06F 21/31, G06Q 10/04, G06Q 10/06, G06Q 10/0631, G06Q 10/0635, G06F 21/62, G06Q 50/22, G06Q 50/26, G08B 21/12, G16H 10/40, G16H 40/20

(54) **METHOD FOR PROVIDING LABORATORY ENVIRONMENT OPTIMIZED FOR USER**
VERFAHREN ZUR BEREITSTELLUNG EINER FÜR DEN BENUTZER OPTIMIERTEN LABORUMGEBUNG
PROCÉDÉ POUR FOURNIR UN ENVIRONNEMENT DE LABORATOIRE OPTIMISÉ POUR UN UTILISATEUR

(30) Priority: 15.05.2020 KR 20200058224
(43) Date of publication of application: 12.01.2022
(73) Proprietor: GTSCIEN CO., LTD., Yuseong-gu, Daejeon 34016 (KR)
(72) Inventor: KANG, Yeon Kyun, Daejeon 34401 (KR); JE, Sung Kwan, Daejeon 35207 (KR)
(74) Representative: Fuchs Patentanwälte Partnerschaft mbB
(86) International application number: PCT/KR2020/006819
(87) International publication number: WO 2021/230410

(56) References cited:
- EP-A1- 3 171 302
- KR-A- 20110 085 350
- KR-A- 20160 112 075
- KR-A- 20160 124 472
- KR-A- 20190 018 788
- KR-B1- 101 746 515

## Description

### CROSS-REFERENCE TO RELATED APPLICATION

This application claims priority to and the benefit of Korean Patent Application No. 10-2020-0058224, filed on May 15, 2020.

### BACKGROUND

### 1. Field of the Invention

The present invention relates to a method of providing a user-optimized laboratory environment.

### 2. Discussion of Related Art

Recently, as interest in the environment, health, and safety (EHS) is growing not only in Korea but also globally, related regulations are also being tightened. Accordingly, research and technology development at the domestic and foreign policy level are accelerating through the enforcement and revision of related laws and regulations for laboratories and occupational safety and health.

Although material safety data sheets (MSDSs) are provided to prevent accidents caused by industrial harmful substances and to enable quick response to the accidents, accidents still occur frequently. Therefore, it is necessary to establish a system for data collection and processing specialized in occupational safety and health.

EP 3 171 302 A1 relates to generating an entry for an electronic laboratory journal using a selected laboratory workflow.

The above description is intended only to help with understanding of the background of the technical scope of the present invention, and therefore it cannot be understood as the content corresponding to the related art known to those skilled in the art of the present invention.

### SUMMARY OF THE INVENTION

The present invention is directed to providing a method of providing a user-optimized laboratory environment in which accidents in a laboratory of a user are prevented. The invention is as set out in the appended claims.

According to an aspect of the present invention, there is provided a method of providing a user-optimized laboratory environment, which includes receiving user location information and user experiment information from a user terminal, receiving location information of laboratory equipment and internal environment information of a laboratory from a plurality of sensors, providing experiment guide information to a user on the basis of the user location information, the user experiment information, the location information of the laboratory equipment, and the internal environment information of the laboratory, and controlling the laboratory equipment on the basis of the user location information, the user experiment information, the location information of the laboratory equipment, and the internal environment information of the laboratory.

The receiving of the user experiment information from the user terminal may include receiving user security level information and information about reagents used by the user for the experiment from the user terminal.

The receiving of the location information of the laboratory equipment from the plurality of sensors may include receiving location information of laboratory equipment including a reagent cabinet, a smart table, a harmful gas purification device, a fume hood, and conditioning equipment from the plurality of sensors.

The receiving of the internal environment information of the laboratory from the plurality of sensors may include receiving information about air quality inside the laboratory, which includes a concentration of a harmful gas generated inside the laboratory and a concentration of a high-risk and highly-volatile harmful gas, and laboratory temperature information from the plurality of sensors.

The providing of the experiment guide information to the user on the basis of the user location information, the user experiment information, the location information of the laboratory equipment, and the internal environment information of the laboratory may include determining whether the user is located in the laboratory on the basis of the user location information and providing internal environment information of the laboratory which is obtained in real time and internal environment information of the laboratory which is predicted during the experiment to the user on the basis of the user experiment information and the internal environment information of the laboratory.

The providing of the experiment guide information to the user on the basis of the user location information, the user experiment information, the location information of the laboratory equipment, and the internal environment information of the laboratory may include determining whether the user is located in the laboratory on the basis of the user location information, providing a material safety data sheet for reagents that are used by the user to the user on the basis of the user experiment information, and providing the experiment guide information to the user so that an experiment by which a high-risk group harmful gas is generated is performed in a fume hood.

The providing of the experiment guide information to the user on the basis of the user location information, the user experiment information, the location information of the laboratory equipment, and the internal environment information of the laboratory may include providing the experiment guide information to the user terminal on the basis of the user location information, the user experiment information, the location information of the laboratory equipment, and the internal environment information of the laboratory.

The providing of the experiment guide information to the user on the basis of the user location information, the user experiment information, the location information of the laboratory equipment, and the internal environment information of the laboratory may include providing the experiment guide information through a display of a smart table in the laboratory on the basis of the user location information, the user experiment information, the location information of the laboratory equipment, and the internal environment information of the laboratory.

The controlling of the laboratory equipment on the basis of the user location information, the user experiment information, the location information of the laboratory equipment, and the internal environment information of the laboratory may include controlling the laboratory equipment including the harmful gas purification device, the fume hood, and the conditioning equipment according to the internal environment information of the laboratory which is predicted during the experiment on the basis of the user experiment information and the internal environment information of the laboratory, and adjusting the air quality in the laboratory and the temperature of the laboratory.

The controlling of the laboratory equipment on the basis of the user location information, the user experiment information, the location information of the laboratory equipment, and the internal environment information of the laboratory may include determining whether an experiment by which a high-risk group harmful gas is generated is performed in a fume hood on the basis of the user location information, the user experiment information, the location information of the laboratory equipment, and the internal environment information of the laboratory, controlling the laboratory equipment including the harmful gas purification device, the fume hood, and the conditioning equipment on the basis of the user location information, the user experiment information, the location information of the laboratory equipment, and the internal environment information of the laboratory, and adjusting the air quality in the laboratory and the temperature of the laboratory.

The controlling of the laboratory equipment on the basis of the user location information, the user experiment information, the location information of the laboratory equipment, and the internal environment information of the laboratory may include determining whether an experiment by which a high-risk group harmful gas is generated is performed in a fume hood using the user location information and location information of the fume hood, controlling the laboratory equipment including the harmful gas purification device, the fume hood, and the conditioning equipment on the basis of the internal environment information of the laboratory, and adjusting the air quality in the laboratory and the temperature of the laboratory.

The controlling of the laboratory equipment on the basis of the user location information, the user experiment information, the location information of the laboratory equipment, and the internal environment information of the laboratory may include controlling a locking device of a reagent cabinet in the laboratory on the basis of the information about the reagents used by the user for the experiment, and adjusting an access region of the user in the reagent cabinet.

According to another aspect of the present invention, there is provided a method of providing a user-optimized laboratory environment, which includes receiving user experiment information from a user terminal, retrieving reference experiment information corresponding to the user experiment information from a memory, receiving internal environment information of a laboratory from a plurality of sensors, and providing experiment guide information including internal environment information of the laboratory which is obtained in real time and internal environment information of the laboratory which is predicted for each time period during the experiment to a user on the basis of the user experiment information, the reference experiment information, and the internal environment information of the laboratory.

The reference experiment information may be experimental information which is previously performed using the same reagent as the reagent used by the user for the experiment and may be accumulated as the experiment is continuously performed.

The providing of the internal environment information of the laboratory which is obtained in real time and the experiment guide information including the internal environment information of the laboratory which is predicted for each time period during the experiment to the user on the basis of the user experiment information, the reference experiment information, and the internal environment information of the laboratory may include analyzing a similarity between the reference experiment information and the user experiment information, and calculating and providing the internal environment information of the laboratory predicted for each time period during the experiment on the basis of the previous environment internal information of the laboratory related to the reference experiment information and the real-time internal environment information of the laboratory.

The internal environment information of the laboratory predicted for each time period during the experiment may be derived by analyzing a similarity between reagent information included in the user experiment information and reagent information included in the reference experiment information, applying the similarity to the previous environment internal information of the laboratory related to the reference experiment information, and then correcting the internal environment information of the laboratory with the real-time internal environment information of the laboratory.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG 1 is a schematic configuration diagram illustrating a system for providing a user-optimized laboratory environment according to an embodiment of the present invention;
FIG 2 is a schematic diagram illustrating a configuration of laboratory equipment of FIG 1 according to an embodiment of the present invention;
FIG 3 is a flowchart illustrating a method of providing a user-optimized laboratory environment according to an embodiment of the present invention; and
FIG 4 is a flowchart illustrating a method of providing a user-optimized laboratory environment according to another embodiment of the present invention.

### DETAILED DESCRIPTION OF EXEMPLARY EMBODIMENTS

In the following description, for purposes of explanation, numerous specific details are introduced to help with understanding various embodiments. It will be apparent, however, that various embodiments may be performed without these specific details or in one or more equivalent manners. In other examples, well-known structures and devices are illustrated in block diagrams in order to avoid unnecessarily obscuring the various embodiments.

In the accompanying drawings, the size or relative size of layers, films, panels, regions, etc. may be exaggerated for clarity of description. Further, like reference numerals indicate like components.

Throughout this specification, when a part is referred to as being "connected" to another part, it includes "directly connected" and "indirectly connected" via an intervening part. However, when it is described that a part is "directly connected" to another part, this will mean that there is no other elements between the part and another part. When the term "at least any one of X, Y, and Z" and "at least any one selected from the group consisting of X, Y, and Z" is used, it should be understood to include one X, one Y, one Z, or any combination of two or more of X, Y, and Z (e.g., XYZ, XYY, YZ, and ZZ). Here, the term "and/or" includes any one or all possible combinations of the components.

Although terms such as first, second, etc. may be used herein to describe various elements, components, regions, layers, and/or sections, such elements, components, regions, layers, and/or or sections are not limited by the terms. These terms are used to distinguish one element, component, region, layer, and/or section from another element, component, region, layer, and/or section. Accordingly, a first element, component, region, layer, and/or section in one embodiment may be referred to as a second element, component, region, layer, and/or section in another embodiment.

The spatially-relative terms such as "below," "beneath," "lower," "above," "upper," etc. may be used herein for ease of description to describe the relationship of one element or feature with another element(s) or feature(s) as illustrated in the drawings. This is only used to indicate the relationship of one component to another component in the drawing and does not mean an absolute position. For example, when the device in the drawings is turned over, elements or features described as "below" or "beneath" other elements or features would then be oriented "above" the other elements or the other features. Therefore, in one embodiment, the term "below" may encompass both an orientation of above and below. In addition, the device may be otherwise oriented (e.g., rotated 90 degrees or in other orientations), and such spatially-relative terms used herein are interpreted accordingly.

The terminology used herein is for the purpose of describing particular embodiments only and is not intended to be limiting to the present invention. Throughout this specification, when a certain part "includes" a certain component, it means that another component may be further included not excluding another component unless otherwise defined. Unless otherwise defined, all terms used herein have the same meaning as commonly understood by one of ordinary skill in the art to which the present invention belongs.

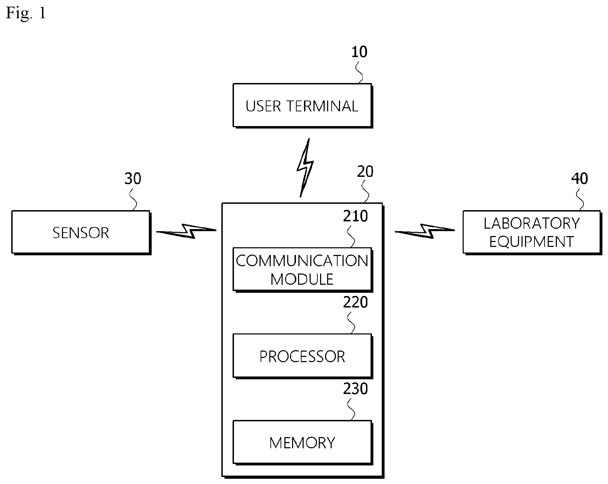

FIG 1 is a schematic configuration diagram illustrating a system for providing a user-optimized laboratory environment according to an embodiment of the present invention.

Referring to FIG 1, a system 20 for providing a user-optimized laboratory environment includes a communication module 210, a processor 220, and a memory 230, is connected to a user terminal 10, a sensor 30, and laboratory equipment 40 through a network, and transmits or receives all pieces of data and information necessary to provide the user-optimized laboratory environment to or from the user terminal 10, the sensor 30, and the laboratory equipment 40. In an embodiment, the system 20 for providing a user-optimized laboratory environment may include only the communication module 210 and the processor 220.

Although a mobile communication terminal which is connected to the network and capable of transmitting or receiving data is described as a representative example of the user terminal 10, the user terminal 10 is not limited to the mobile communication terminal and may include any information communication device and include one of various terminals such as multimedia terminals, wired/wireless terminals, fixed-type terminals, Internet Protocol (IP) terminals, and the like. Further, the user terminal 10 may include a mobile terminal having various mobile communication specifications, such as a mobile phone, a smartphone, a desktop computer, a tablet personal computer (PC), a notebook computer, a virtual reality (VR) device, an augmented reality (AR) device, a netbook computer, a portable multimedia player (PMP), a mobile Internet device (MID), a server, an information communication device, or the like.

As the network, various types of communication networks may be used and, for example, wireless communication methods, such as wireless local area network (WLAN), Bluetooth, Wi-Fi, WiBro, Worldwide Interoperability for Microwave Access (WiMAX), High Speed Downlink Packet Access (HSDPA), and the like, or wired communication methods, such as Ethernet, x digital subscriber line (xDSL) (asymmetric digital subscriber line (ADSL), very high-speed digital subscriber line (VDSL)), hybrid fiber-coaxial (HFC), fiber to the curb (FTTC), fiber to the home (FTTH), and the like, may be used. Meanwhile, the network is not limited to the communication methods described above and may include any type of communication method that is widely known or to be developed in the future in addition to the above-described communication methods. In an embodiment, the user terminal 10 and the system 20 for providing a user-optimized laboratory environment may transmit or receive data to or from each other through short-range wireless communication, for example, Bluetooth, a radio frequency (RF) wireless method, serial communication such as Inter-Integrated Circuit (I²C), or the like.

The sensor 30 includes a plurality of sensors, detects an internal environment of a laboratory, and transmits information about the internal environment of the laboratory to the system 20 for providing a user-optimized laboratory environment. In an embodiment, the sensor 30 may include a temperature sensor, a humidity sensor, a gas sensor, a flow velocity sensor, a total volatile organic compounds (TVOCs) concentration sensor, and a Global Positioning System (GPS) sensor. In an embodiment, the sensor 30 may be embedded in the laboratory equipment 40.

The laboratory equipment 40 is controlled by the system 20 for providing a user-optimized laboratory environment to provide an optimized laboratory environment to a user. In an embodiment, the laboratory equipment 40 may include a reagent cabinet, a smart table, a harmful gas purification device, a fume hood, and conditioning equipment.

The system 20 for providing a user-optimized laboratory environment includes the communication module 210 for communication with the user terminal 10, the sensor 30, and the laboratory equipment 40 through the network, the processor 220, and the memory 230 for storing reference experiment information.

The communication module 210 is connected to the user terminal 10, the sensor 30, and the laboratory equipment 40 through the network to transmit or receive all pieces of data and information necessary to provide the user-optimized laboratory environment. In an embodiment, the communication module 210 may include at least one of a mobile communication module, a wireless Internet module, and a short-range communication module.

The processor 220 receives user location information and user experiment information from the user terminal 10, receives location information of the laboratory equipment and internal environment information of the laboratory from the sensor 30, and then provides experiment guide information to the user through the communication module 210 on the basis of the user location information, the user experiment information, the location information of the laboratory equipment, and the internal environment information of the laboratory. In an embodiment, the processor 220 may provide the experiment guide information to the user terminal 10. In another embodiment, the processor 220 may provide the experiment guide information to the laboratory equipment 40. Further, the processor 220 controls the laboratory equipment 40 on the basis of the user location information, the user experiment information, the location information of the laboratory equipment, and the internal environment information of the laboratory.

The memory 230 is configured to store all pieces of data and information necessary to provide the user-optimized laboratory environment. In an embodiment, the memory 230 may be configured to store the reference experiment information.

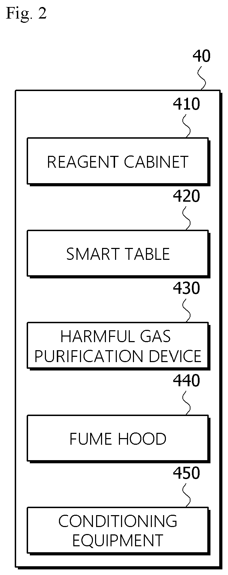

FIG 2 is a schematic diagram illustrating a configuration of the laboratory equipment of FIG 1 according to the embodiment of the present invention.

Referring to FIG 2, the laboratory equipment 40 may include a reagent cabinet 410, a smart table 420, a harmful gas purification device 430, a fume hood 440, and conditioning equipment 450.

The reagent cabinet 410 is equipment for storing reagents required for experiments. The system 20 for providing a user-optimized laboratory environment may control a locking device of the reagent cabinet 410 or adjust an access region in the reagent cabinet 410.

The smart table 420 is equipment on which the user mainly conducts experiments and may include a display. In an embodiment, the experiment guide information may be provided to the user through the display of the smart table 420.

The harmful gas purification device 430 is equipment for purifying a harmful gas detected by the sensor in the laboratory using a filter and may be controlled by the system 20 for providing a user-optimized laboratory environment.

The fume hood 440 is equipment for protecting the user from exposure to volatile chemicals and provides a protection function against fire, chemical reaction, odor, and leakage and discharges dust, smoke, and harmful gases in the laboratory. In an embodiment, the fume hood 440 may be controlled by the system 20 for providing a user-optimized laboratory environment.

The conditioning equipment 450 is equipment for controlling air quality in the laboratory and may include an air conditioner and a ventilation system. In an embodiment, the conditioning equipment 450 may be controlled by the system 20 for providing a user-optimized laboratory environment.

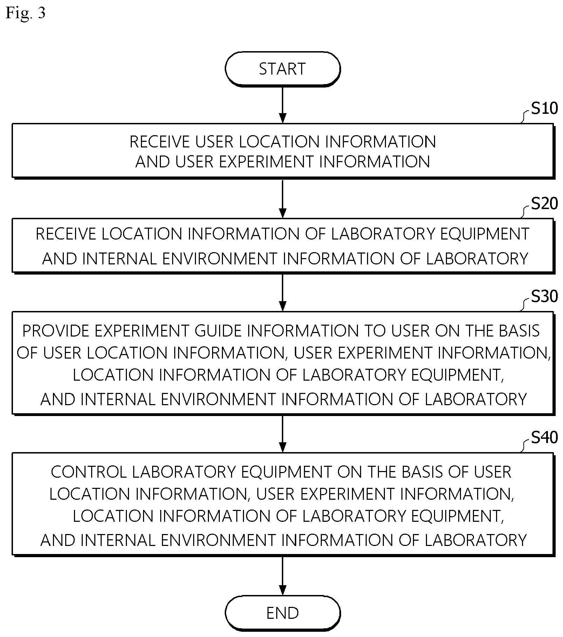

FIG 3 is a flowchart illustrating a method of providing a user-optimized laboratory environment according to an embodiment of the present invention.

Referring to FIG 3, a system for providing a user-optimized laboratory environment receives user location information and user experiment information from a user terminal (S10). In an embodiment, the system for providing a user-optimized laboratory environment may receive user security level information and information about reagents used by the user for an experiment from the user terminal.

The system for providing a user-optimized laboratory environment receives location information of laboratory equipment and internal environment information of a laboratory from a plurality of sensors (S20). In an embodiment, the system for providing a user-optimized laboratory environment may receive location information of laboratory equipment including a reagent cabinet, a smart table, a harmful gas purification device, a fume hood, and conditioning equipment from the plurality of sensors. In another embodiment, the system for providing a user-optimized laboratory environment may receive information about air quality inside the laboratory, which includes a concentration of a harmful gas generated inside the laboratory and a concentration of a high-risk and highly-volatile harmful gas, and laboratory temperature information from the plurality of sensors.

The system for providing a user-optimized laboratory environment provides experiment guide information to the user on the basis of the user location information, the user experiment information, the location information of the laboratory equipment, and the internal environment information of the laboratory (S30). In an embodiment, the system for providing a user-optimized laboratory environment may determine whether the user is located in the laboratory on the basis of the user location information and provide internal environment information of the laboratory which is obtained in real time and internal environment information of the laboratory which is predicted during the experiment to the user on the basis of the user experiment information and the internal environment information of the laboratory. In another embodiment, the system for providing a user-optimized laboratory environment may determine whether the user is located in the laboratory on the basis of the user location information, provide a material safety data sheet for reagents that are used by the user to the user on the basis of the user experiment information, and provide the experiment guide information so as to perform an experiment, in which a high-risk group harmful gas is generated, in the fume hood.

The system for providing a user-optimized laboratory environment according to the embodiment of the present invention may provide the experiment guide information to the user terminal on the basis of the user location information, the user experiment information, the location information of the laboratory equipment, and the internal environment information of the laboratory. In an embodiment, the system for providing a user-optimized laboratory environment may provide the experiment guide information through a display of the smart table in the laboratory on the basis of the user location information, the user experiment information, the location information of the laboratory equipment, and the internal environment information of the laboratory.

The system for providing a user-optimized laboratory environment controls the laboratory equipment on the basis of the user location information, the user experiment information, the location information of the laboratory equipment, and the internal environment information of the laboratory (S40). In an embodiment, the system for providing a user-optimized laboratory environment may control the laboratory equipment including the harmful gas purification device, the fume hood, and the conditioning equipment according to the internal environment information of the laboratory which is predicted during the experiment on the basis of the user experiment information and the internal environment information of the laboratory to adjust air quality in the laboratory and a temperature of the laboratory. In an embodiment, the system for providing a user-optimized laboratory environment may determine whether the experiment, in which the high-risk group harmful gas is generated, is performed in the fume hood on the basis of the user location information, the user experiment information, the location information of the laboratory equipment, and the internal environment information of the laboratory, and may control the laboratory equipment including the harmful gas purification device, the fume hood, and the conditioning equipment on the basis of the user location information, the user experiment information, the location information of the laboratory equipment, and the internal environment information of the laboratory to adjust the air quality in the laboratory and the temperature of the laboratory.

The system for providing a user-optimized laboratory environment according to the embodiment of the present invention may determine whether the experiment, in which the high-risk group harmful gas is generated, is performed in the fume hood using the user location information and location information of the fume hood and may control the laboratory equipment including the harmful gas purification device, the fume hood, and the conditioning equipment on the basis of the internal environment information of the laboratory to adjust the air quality in the laboratory and the temperature of the laboratory. In an embodiment, the system for providing a user-optimized laboratory environment may control a locking device of the reagent cabinet in the laboratory on the basis of the information about the reagents used by the user for the experiment to adjust an access region of the user in the reagent cabinet.

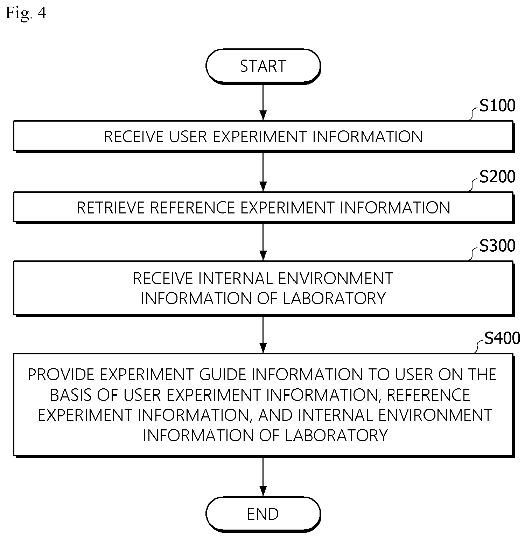

FIG 4 is a flowchart illustrating a method of providing a user-optimized laboratory environment according to another embodiment of the present invention.

Referring to FIG 4, the system for providing a user-optimized laboratory environment receives user experiment information from a user terminal (S100) and retrieves reference experiment information corresponding to the user experiment information from a memory (S200). In an embodiment, the reference experiment information is information about an experiment which is previously performed using the same reagent as the reagent used by the user for the experiment and may be accumulated as the experiment is continuously performed.

The system for providing a user-optimized laboratory environment receives internal environment information of a laboratory from a plurality of sensors (S300) and provides experiment guide information including internal environment information of the laboratory which is obtained in real time and internal environment information of the laboratory which is predicted for each time period during the experiment to the user on the basis of the user experiment information, the reference experiment information, and the internal environment information of the laboratory (S400). In an embodiment, the system for providing a user-optimized laboratory environment may analyze a similarity between the reference experiment information and the user experiment information and calculate and provide the internal environment information of the laboratory predicted for each time period during the experiment on the basis of the previous environment internal information of the laboratory related to the reference experiment information and the real-time internal environment information of the laboratory. In an embodiment, the internal environment information of the laboratory predicted for each time period during the experiment may be derived by analyzing a similarity between reagent information included in the user experiment information and reagent information included in the reference experiment information, applying the similarity to the previous environment internal information of the laboratory related to the reference experiment information, and then correcting the internal environment information of the laboratory with the real-time internal environment information of the laboratory.

According to the embodiments of the present invention as described above, the method of providing the user-optimized laboratory environment according to the embodiment of the present invention may prevent accidents in a laboratory of a user by controlling laboratory equipment on the basis of user information and laboratory environment information. Further, by providing internal environment information of the laboratory predicted during an experiment to the user, it is possible to contribute to the user's activities for laboratory safety and health.

As described above, while the present invention has been described with reference to the specific details of the detailed components and the specific embodiments and drawings, these are only examples to facilitate overall understanding of the present invention, and the present invention is not limited thereto. It will be understood by one of ordinary skill in the art to which the present invention belongs that various modifications and alterations may be made.

## Claims

1. A method of providing a user-optimized laboratory environment, the method performed by a system (20) and comprising:
receiving (S100) user experiment information from a user terminal (10);
retrieving (S200) reference experiment information corresponding to the user experiment information from a memory;
receiving (S300) internal environment information of a laboratory from a plurality of sensors (30); and
providing (S400) experiment guide information including internal environment information of the laboratory which is obtained in real time and internal environment information of the laboratory which is predicted for each time period during the experiment to a user on the basis of the user experiment information, the reference experiment information, and the internal environment information of the laboratory, wherein the reference experiment information is experimental information which is previously performed using the same reagent as the reagent used by the user for the experiment and is accumulated as the experiment is continuously performed.

2. The method of claim 1, wherein the providing (S400) of the internal environment information of the laboratory which is obtained in real time and the experiment guide information including the internal environment information of the laboratory which is predicted for each time period during the experiment to the user on the basis of the user experiment information, the reference experiment information, and the internal environment information of the laboratory includes:
analyzing a similarity between the reference experiment information and the user experiment information; and
calculating and providing the internal environment information of the laboratory predicted for each time period during the experiment on the basis of the previous environment internal information of the laboratory related to the reference experiment information and the real-time internal environment information of the laboratory.

3. The method of claim 1, wherein the internal environment information of the laboratory predicted for each time period during the experiment is derived by analyzing a similarity between reagent information included in the user experiment information and reagent information included in the reference experiment information, applying the similarity to the previous environment internal information of the laboratory related to the reference experiment information, and then correcting the internal environment information of the laboratory with the real-time internal environment information of the laboratory.

## Patentansprüche

1. Verfahren zum Bereitstellen einer benutzeroptimierten Laborumgebung, wobei das Verfahren durch ein System (20) ausgeführt wird und aufweist:
Empfangen (S100) von Benutzer-Experimentinformation von einem Benutzerendgerät (10);
Abrufen (S200) von der Benutzer-Experimentinformation entsprechender Referenz-Experimentinformation aus einem Speicher;
Empfangen (S300) von Information über Umgebungsbedingungen innerhalb eines Labors von einer Vielzahl von Sensoren (30); und
Bereitstellen (S400) von Experimentanleitungsinformation, die in Echtzeit erhaltene Information über Umgebungsbedingungen innerhalb des Labors und für jeden Zeitraum während des Experiments vorausgesagte Information über Umgebungsbedingungen innerhalb des Labors enthält, für einen Benutzer auf der Grundlage der Benutzer-Experimentinformation, der Referenz-Experimentinformation und der Information über Umgebungsbedingungen innerhalb des Labors, wobei die Referenz-Experimentinformation Experimentinformation ist, die zuvor unter Verwendung des gleichen Reagenzes erhalten wurde, das der Benutzer für das Experiment verwendet, und die akkumuliert wird, während das Experiment kontinuierlich ausgeführt wird.

2. Verfahren nach Anspruch 1, wobei das Bereitstellen (S400) der in Echtzeit erhaltenen Information über Umgebungsbedingungen innerhalb des Labors und der Experimentanleitungsinformation, die die für jeden Zeitraum während des Experiments vorausgesagte Information über Umgebungsbedingungen innerhalb des Labors enthält, für den Benutzer auf der Grundlage der Benutzer-Experimentinformation, der Referenz-Experimentinformation und der Information über Umgebungsbedingungen innerhalb des Labors aufweist:
Analysieren einer Ähnlichkeit zwischen der Referenz-Experimentinformation und der Benutzer-Experimentinformation; und
Berechnen und Bereitstellen der für jeden Zeitraum während des Experiments vorausgesagten Information über Umgebungsbedingungen innerhalb des Labors auf der Grundlage der vorangehenden Information über Umgebungsbedingungen innerhalb des Labors, die mit der Referenz-Experimentinformation in Beziehung steht, und der in Echtzeit erhaltenen Information über Umgebungsbedingungen innerhalb des Labors.

3. Verfahren nach Anspruch 1, wobei die für jeden Zeitraum während des Experiments vorhergesagte Information über Umgebungsbedingungen innerhalb des Labors hergeleitet wird durch Analysieren einer Ähnlichkeit zwischen der in der Benutzer-Experimentinformation enthaltenen Reagenzinformation und der in der Referenz-Experimentinformation enthaltenen Reagenzinformation, Anwenden der Ähnlichkeit auf die vorhergehende Information über Umgebungsbedingungen innerhalb des Labors, die mit der Referenz-Experimentinformation in Beziehung steht, und anschließendes Korrigieren der Information über Umgebungsbedingungen innerhalb des Labors mit der in Echtzeit erhaltenen Information über Umgebungsbedingungen innerhalb des Labors.

## Revendications

1. Procédé de fourniture d'un environnement de laboratoire optimisé pour l'utilisateur, le procédé étant exécuté par un système (20) et comprenant :
la réception (S100) d'informations d'expérience d'utilisateur en provenance d'un terminal utilisateur (10) ;
la récupération (S200) d'informations d'expérience de référence correspondant aux informations d'expérience d'utilisateur à partir d'une mémoire ;
la réception (S300) d'informations d'environnement interne d'un laboratoire en provenance d'une pluralité de capteurs (30) ; et
la fourniture (S400) d'informations de guide d'expérience comprenant des informations d'environnement interne du laboratoire qui sont obtenues en temps réel et des informations d'environnement interne du laboratoire qui sont prédites pour chaque période de temps pendant l'expérience à un utilisateur sur la base des informations d'expérience d'utilisateur, des informations d'expérience de référence et des informations d'environnement interne du laboratoire, dans lequel les informations d'expérience de référence sont des informations expérimentales qui sont préalablement exécutées en utilisant le même réactif que le réactif utilisé par l'utilisateur pour l'expérience et sont accumulées à mesure que l'expérience est exécutée de manière continue.

2. Procédé selon la revendication 1, dans lequel la fourniture (S400) des informations d'environnement interne du laboratoire qui sont obtenues en temps réel et des informations de guide d'expérience comprenant les informations d'environnement interne du laboratoire qui sont prédites pour chaque période de temps pendant l'expérience à l'utilisateur sur la base des informations d'expérience d'utilisateur, des informations d'expérience de référence et des informations d'environnement interne du laboratoire, comprend :
l'analyse d'une similarité entre les informations d'expérience de référence et les informations d'expérience d'utilisateur ; et
le calcul et la fourniture des informations d'environnement interne du laboratoire prédites pour chaque période de temps pendant l'expérience sur la base des informations d'environnement interne précédentes du laboratoire liées aux informations d'expérience de référence et des informations d'environnement interne en temps réel du laboratoire.

3. Procédé selon la revendication 1, dans lequel les informations d'environnement interne du laboratoire prédites pour chaque période de temps pendant l'expérience sont dérivées en analysant une similarité entre les informations de réactif incluses dans les informations d'expérience d'utilisateur et les informations de réactif incluses dans les informations d'expérience de référence, en appliquant la similarité aux informations d'environnement interne précédentes du laboratoire liées aux informations d'expérience de référence, puis en corrigeant les informations d'environnement interne du laboratoire avec les informations d'environnement interne en temps réel du laboratoire.
